# EUROPEAN PATENT APPLICATION

(11) **EP 4 374 908 A1**
(43) Date of publication of application: **29.05.2024**
(21) Application number: 21951055.9
(22) Date of filing: 29.12.2021
(51) Int. Cl.: A61M 37/00, A61F 13/02, A61F 13/00

(54) **METHOD FOR MANUFACTURING FUNCTIONAL MIXTURE-COATED SKIN ATTACHMENT THIN FILM AND SKIN-ATTACHMENT THIN FILM MANUFACTURED BY USING SAME**

(30) Priority: 21.07.2021 KR 20210095534
(71) Applicant: Korea Institute of Machinery & Materials, Daejeon 34103 (KR); CRScube America, Inc., Germantown, Maryland 20876 (US)
(72) Inventor: JEONG, Jun-ho, Daejeon 34103 (KR); JEON, Sohee, Daejeon 34103 (KR); HWANG, Soon-Hyoung, Daejeon 34103 (KR); KANG, Hyeok Jung, Daejeon 34103 (KR); KIM, Ki Don, Seoul 04170 (KR)
(74) Representative: Germain Maureau
(86) International application number: PCT/KR2021/020224
(87) International publication number: WO 2023/003107

(57) **Abstract**

Provided is a method of manufacturing a skin attachable thin film coated with a functional mixture, the method including: preparing a microstructured thin film including a base thin film and at least one microstructure protruding from the base thin film; and coating a functional mixture on the microstructure excluding a region of the base film of the microstructured thin film, wherein in the coating the mixture, the functional mixture is irregularly coated on the at least one microstructure.

## Description

### [Technical Field]

The present disclosure relates to a method of manufacturing a skin attachable thin film coated with a functional mixture and a skin attachable thin film manufactured using the same, and more particularly, to a method of manufacturing a skin attachable thin film to quickly coat the functional mixture on a microstructure, and a skin attachable thin film manufactured using the same.

### [Background Art]

In generally, most medicines may be orally administered. However, a certain medicine, especially a protein or peptide medicine, may not be effectively adsorbed into a human body by this method due to its intense degradation in the gastrointestinal tract, its poor absorption from an intestinal cell membrane, and/or an interruption of its first-pass by the liver.

Another medicine administration technique may be parenteral injection using a standard syringe or a catheter. Needle injection may cause a needle phobia, a substantial pain, and a local damage to the skin in many patients. Retrieval of a body fluid, such as blood, for a diagnostic purpose may raise a similar concern. In addition, the needle injection may not be ideal for a regular medicine delivery or a regular diagnosis.

Still another medicine delivery technology may use a way of forming a microstructure on a thin film and attaching the same to the skin, thereby delivering the medicine into the skin through the microstructure. This way may include a method of manufacturing the microstructure itself by drying the same with the medicine. However, it may be difficult to immediately deliver the medicine because it takes up to several hours for the microstructure inserted into the transdermis to be completely decomposed to deliver the medicine.

A technique for coating the medicine on the microstructure has been developed as another method. However, this technique has a limitation in mass production because a significant amount of medicine is discarded during a coating process, and a complex process is required for coating each microstructure with a certain amount of medicine.

### [Disclosure]

### [Technical Problem]

The present disclosure attempts to provide a method of manufacturing a skin attachable thin film in which a functional mixture is coated on a region of a microstructure protruding from a base thin film through a simple process, and a skin attachable thin film manufactured using the same.

The present disclosure also attempts to minimize a manufacturing cost of the skin attachable thin film by minimizing the functional mixture discarded during a coating process.

The present disclosure also attempts to provide a method of manufacturing a skin attachable thin film in which an amount of a functional mixture injected into the skin may be relatively accurately controlled, and a skin attachable thin film manufactured using the same.

### [Technical Solution]

According to an embodiment, provided is a method of manufacturing a skin attachable thin film coated with a functional mixture, the method including: a step of preparing a microstructured thin film including at least one microstructure protruding from a base thin film; and a step of coating a functional mixture on the microstructure excluding a region of the base film of the microstructured thin film, wherein in the step of coating the mixture, the functional mixture is irregularly coated on a partial unspecified region of the at least one microstructure.

In addition, the method includes: the step of preparing the microstructured thin film including the at least one microstructure protruding from the base thin film; and the step of coating a functional mixture on the microstructure excluding the region of the base film of the microstructured thin film, and may further include a step of adjusting a spray angle of the functional mixture to be coated on the microstructure before the step of coating the mixture is performed.

Here, the method may further include a step of coating a binder material onthe microstructure for increasing an adhesive strength between the functional mixture and the microstructure, before the step of coating the mixture is performed.

In addition, in the step of coating the mixture, the functional mixture may have a droplet structure and is sprayed on the microstructure.

Meawhile, in the step of coating the mixture, the functional mixture may have a fiber structure and is sprayed on the microstructure.

In addition, the microstructured thin film may include any one of an insoluble material, a low solubility material, and a biodegradable material, or a mixture of the insoluble material, the low solubility material, and the biodegradable material.

Here, the step of preparing the thin film may include: a step of preparing a mold having a structure corresponding to a shape of the microstructure to be formed; a step of coating a thin film material, which is a material of the microstructured thin film, on the mold; a step of molding the microstructured thin film by pressing and deforming the thin film material to have the structure formed in the mold as a pressing unit is moved toward the mold; and a step of acquiring the microstructured thin film by separating the microstructured thin film from the mold.

In addition, the step of preparing the thin film may include: a step of preparing a mold having a structure corresponding to a shape of the microstructure to be formed; a step of coating a thin film material, which is a material of the microstructured thin film, on the mold; a step of molding the microstructured thin film as the thin film material coated on the mold is dried; and a step of acquiring the microstructured thin film by separating the microstructured thin film from the mold.

In addition, the step of preparing the thin film may include: a step of preparing a mold having a structure corresponding to a shape of the microstructure to be formed; a step of coating a thin film material, which is a material of the microstructured thin film, on the mold; a step of molding the microstructured thin film by curing the thin film material coated on the mold by emitting heat or light to the thin film material; and a step of acquiring the microstructured thin film by separating the microstructured thin film from the mold, wherein in the step of molding the microstructured thin film, light emitted to the thin film material is any one of ultraviolet light, visible light, and infrared light.

In addition, the step of preparing the thin film may include: a step of manufacturing the thin film plate having a flat plate shape by using a thin film material, which is a material of the microstructured thin film; and a step of cutting and bending a region of the thin film plate that is pressed by a concave-convex part as a pressing unit disposed above the thin film plate and having the concave-convex part formed toward the thin film plate is moved toward the thin film plate, wherein in the step of cutting and bending, the region of the thin film plate that is pressed and bent by the concave-convex part is the microstructure.

Meawhile, in the step of coating the mixture, the functional mixture coated on the microstructure may be irregularly coated on an unspecified region of the microstructure.

In addition, the method includes: the step of preparing the microstructured thin film including at least one microstructure protruding from the base thin film; and the step of coating the functional mixture on the microstructure excluding the region of the base film of the microstructured thin film, wherein the step of preparing the thin film includes a step of forming the microstructure for a distance between the two adjacent microstructures in a direction perpendicular to a spray direction of the functional mixture to be smaller than a diameter of a droplet of the functional mixture, based on a cross section of the microstructure that corresponds to a certain height from a lower part of the microstructure that is connected to the base thin film.

According to another embodiment, provided is a skin attachable thin film, the film including: a microstructured thin film including a base thin film and at least one microstructure protruding from the base thin film; a coating layer having a functional mixture partially coated on the at least one microstructure, and irregularly coated on an unspecified region of the microstructure excluding a region of the base film; and a binder material coated on a surface of the microstructure to increase an adhesive strength between the functional mixture and the microstructure.

According to still another embodiment, provided is a skin attachable thin film, wherein the film is a microstructured thin film having a functional mixture sprayed and coated thereon, the microstructured thin film includes a base thin film and a plurality of microstructures protruding from the base thin film, and a distance between the two adjacent microstructures in a direction perpendicular to a spray direction of the functional mixture is smaller than a diameter of a droplet of the functional mixture, based on a cross section of the microstructure that corresponds to a certain height from each bottom of the plurality of microstructures.

Here, the microstructure may have one vertex, and include at least one ultramicrostructure protruding therefrom.

In addition, a height of the at least one protruding ultramicrostructure may be 1/3 or more of the height of the microstructure based on the base thin film.

In addition, the ultramicrostructure may have a structure for preventing the functional mixture from flowing down along a surface of the microstructure when the functional mixture is coated on the microstructure.

Meawhile, the microstructured thin film may include at least one microstructure to prevent the functional mixture from being in contact with the base thin film when the functional mixture is coated on the microstructure.

In addition, the microstructured thin film may have a distance between the microstructures that is measured based on the vertex of the microstructure, which is longer than a length of a lower surface of the microstructure, and shorter than the height of the microstructure that is measured based on the base thin film to prevent the functional mixture from being in contact with the base thin film when the functional mixture is coated on the microstructure.

Here, the microstructured thin film may include a plurality of nanomicro structures each having a size smaller than that of the microstructure and disposed between the one or more microstructures to prevent the functional mixture from being in contact with the base thin film when the functional mixture is coated on the microstructure.

Meawhile, in the step of preparing the thin film, a nanomicro thin film including a plurality of nanomicro structures each having a size smaller than that of the microstructure may be prepared, and in the step of coating the mixture, the functional mixture may be coated on the microstructure excluding the region of the base film of the microstructured thin film by coating the functional mixture on the nanomicro structure, and disposing the microstructured thin film and the nanomicro thin film to face each other to thus bring the functional mixture into contact with the microstructure.

### [Advantageous Effects]

The method of manufacturing a skin attachable thin film coated with the functional mixture and the skin attachable thin film manufactured using the same according to the present disclosure may have the following effects.

First, the functional mixture may be coated on the region of the microstructure protruding from the base thin film through the simple process.

Second, the manufacturing cost of the skin attachable thin film may be minimized by minimizing the functional mixture discarded during the coating process.

Third, the amount of the functional mixture injected into the skin may be relatively accurately controlled.

### [Description of the Drawings]

FIG. 1 is a view showing a first example and a second example of steps of a method of manufacturing a skin attachable thin film coated with a functional mixture according to the present disclosure.
FIG. 2 is a view showing a step of preparing the thin film according to an embodiment of the present disclosure.
FIGS. 3 and 4 are views showing modifications of a step of preparing the thin film according to an embodiment of the present disclosure.
FIG. 5 is a view showing a step of preparing the thin film in another example of the present disclosure.
FIG. 6 is a view showing a step of coating the mixture according to an embodiment of the present disclosure.
FIG. 7 is a view showing a step of coating the mixture in another example of the present disclosure.
FIG. 8 is a view showing a step of coating the mixture in still another example of the present disclosure.
FIG. 9 is a view showing a first example of a step of adjusting the angle according to an embodiment of the present disclosure.
FIG. 10 is a view showing a second example of a step of adjusting the angle according to an embodiment of the present disclosure.
FIGS. 11 to 14 are views showing microstructures in various examples of the present disclosure.
FIGS. 15 and 16 are views showing microstructured thin films in various examples of the present disclosure.
FIG. 17 is a view for explaining a method of manufacturing a skin attachable thin film in a third example of the present disclosure.

### [Mode for Invention]

Hereinafter, the description describes embodiments of the present disclosure for specifically implementing objects of the present disclosure with reference to the accompanying drawings. In describing the embodiments, the same components are denoted by the same names and the same reference numerals, and the following description omits an additional description thereof.

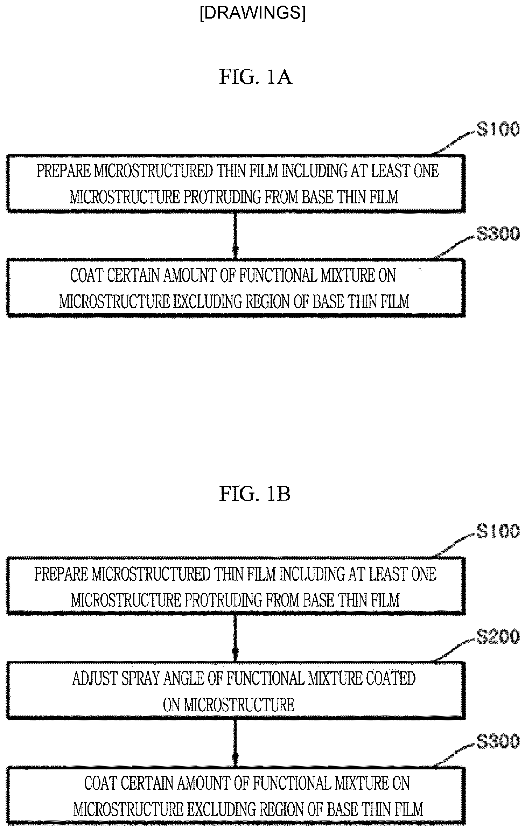

FIG. 1 is a view showing a method of manufacturing a skin attachable thin film coated with a functional mixture according to an embodiment of the present disclosure. FIG. 1A shows a manufacturing method in a first example, and FIG. 1B shows a manufacturing method in a second example.

As shown in FIG. 1A, the method of manufacturing a skin attachable thin film in the first example of the present disclosure may include a step of preparing the thin film (S100) and a step of coating the mixture (S300).

In the step of preparing the thin film (S100), a microstructured thin film including at least one microstructure protruding from a base thin film may be prepared. The microstructure may be formed in the microstructured thin film, for example, in the form of an N X N matrix.

A material included in the microstructured thin film, that is, a thin film material 100B may include any one of an insoluble material, a low-solubility material and a biodegradable material, or a mixture thereof, and examples of the step of preparing the thin film (S100) are described below.

The microstructure may have a cone shape, and the microstructure is not limited to this shape. The microstructure may have a polygonal cross-section, and have an inclined cone shape or a bent shape to facilitate its transdermal insertion. In addition, a region of the microstructure that has the largest area among those of the microstructures, that is, a region connected to the base thin film, may preferably have diameter of several hundred um to several mm.

The step of coating the mixture (S300) may be a step of coating the functional mixture on the microstructure, and the functional mixture may be coated on at least a partial region of the microstructure. The region coated with the functional mixture may be limited to the region of the microstructure that is inserted into the skin among its all regions, excluding a region of the base film.

According to an embodiment of the present disclosure, a step of coating a binder material may be optionally performed before the step of coating the mixture (S300) is performed. The binder material may be a material to increase an adhesive strength between the functional mixture and the microstructure, and the functional mixture may be coated after the binder material is coated. In some examples, the binder material may be mixed with the functional mixture and coated together in the step of coating the mixture (S300).

FIG. 1B shows the method of manufacturing a skin attachable thin film coated with a functional mixture in the second example of the present disclosure. The method may further include a step of adjusting an angle (S200) of adjusting the spray angle of the functional mixture coated on the microstructure before the step of coating the mixture (S300) is performed. The step of adjusting the angle (S200) is described below.

### Step of preparing thin film

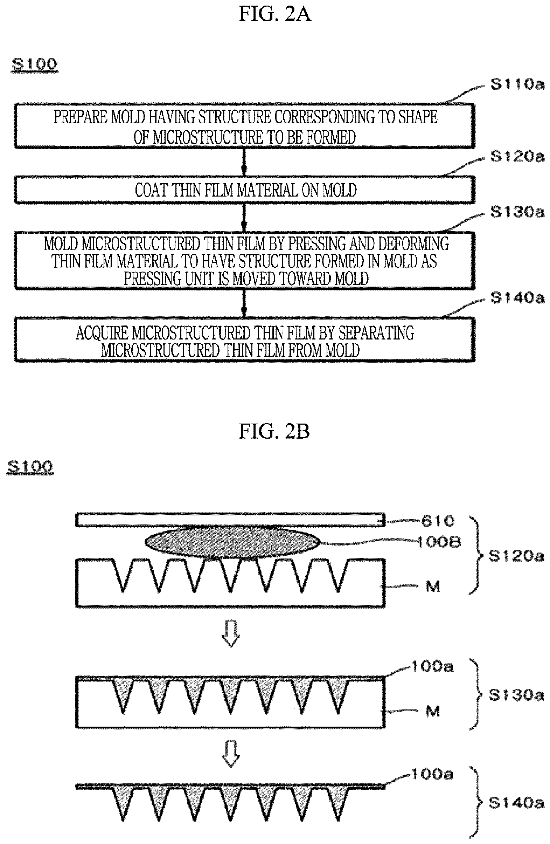

Hereinafter, the description describes the step of preparing the thin film (S100) in various examples of the present disclosure with reference to FIGS. 2 to 5.

FIG. 2 is a view showing the step of preparing the thin film (S100) according to an embodiment of the present disclosure.

As shown in FIG. 2, the step of preparing the thin film (S100) may include: a step of preparing a mold (S110a); a step of coating the thin film material (S120a); a step of molding the thin film (S130a); and a step of acquiring the thin film (S104a).

In the step of preparing the mold (S110a), a mold M having a structure corresponding to the shape of the microstructure to be formed may be prepared.

In the step of coating the thin film material (S120a), the thin film material 100B may be coated on the mold M. Next, in the step of molding the thin film (S130a), a microstructured thin film 100a may be molded by pressing and deforming the thin film material 100B to have the structure formed in the mold M as a pressing unit is moved toward the mold.

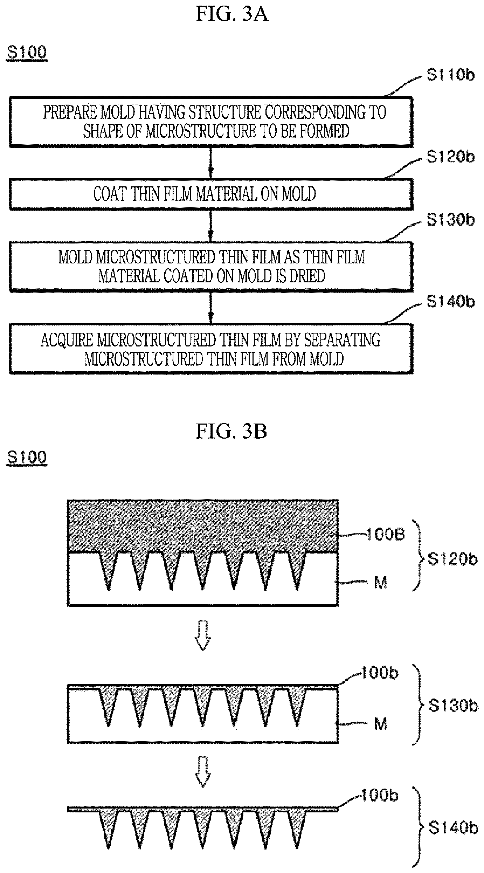

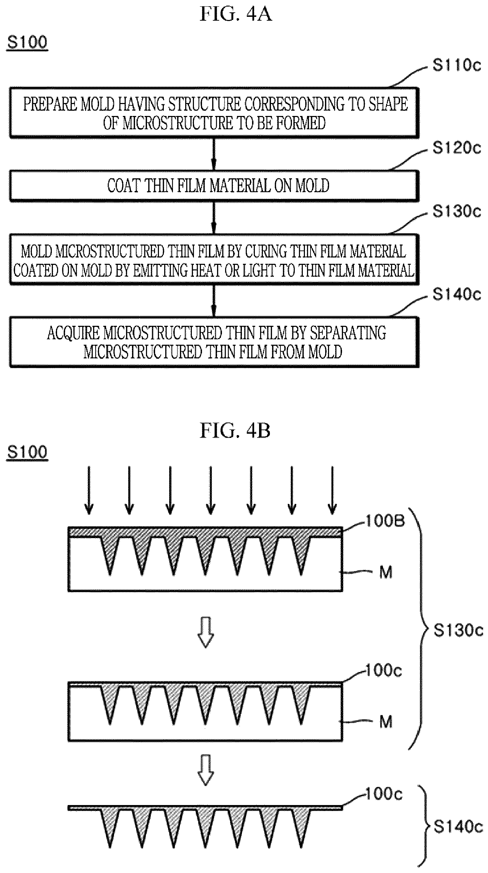

The step of molding the thin film (S130a) may be implemented in various modifications. As shown in FIG. 3, a microstructured thin film 100b may be molded (S130b) as the thin film material 100B coated on the mold M is dried. Alternatively, as shown in FIG. 4, a microstructured thin film 100c may be molded by curing the thin film material 100B coated on the mold M by emitting heat or light such as ultraviolet light, visible light, infrared light, or the like to the thin film material 100B (S130c).

In the step of acquiring the thin film (S104a), the microstructured thin film 100a may be acquired by separating the microstructured thin film 100a from the mold M.

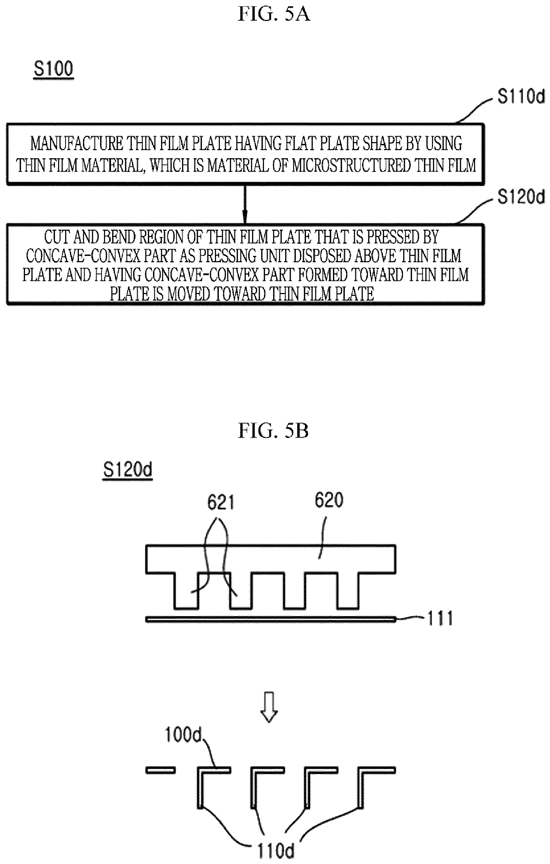

FIG. 5 is a view showing a step of preparing the thin film (S100) in another example of the present disclosure.

As shown in FIG. 5, the step of preparing the thin film (S100) may include: a step of manufacturing a thin film plate (S110d); and a pressing step (S120d).

In the step of manufacturing the thin film plate (S110d), a thin film plate 111 having a flat plate shape may be manufactured by using the thin film material.

In the pressing step (S120d), a region of the thin film plate 111 that is pressed by a concave-convex part 621 may be cut and bent as a pressing unit 620 disposed above the thin film plate 111 and having the concave-convex part 621 formed toward the thin film plate 111 is moved toward the thin film plate 111.

Here, in the pressing step (S120d), a region 110d of the thin film plate 111 that is pressed and bent by the concave-convex part 621 may correspond to the microstructure and a region 100d that is not pressed by the concave-convex part 621 may correspond to the base thin film.

As described above, in the present disclosure, the microstructured thin film may be manufactured by one of various examples of the step of preparing the thin film (S100).

Referring back to FIG. 1, in the step of coating the mixture (S300), the functional mixture may be coated on a microstructure 120, and the functional mixture may be irregularly coated on at least a partial region of at least one microstructure 120.

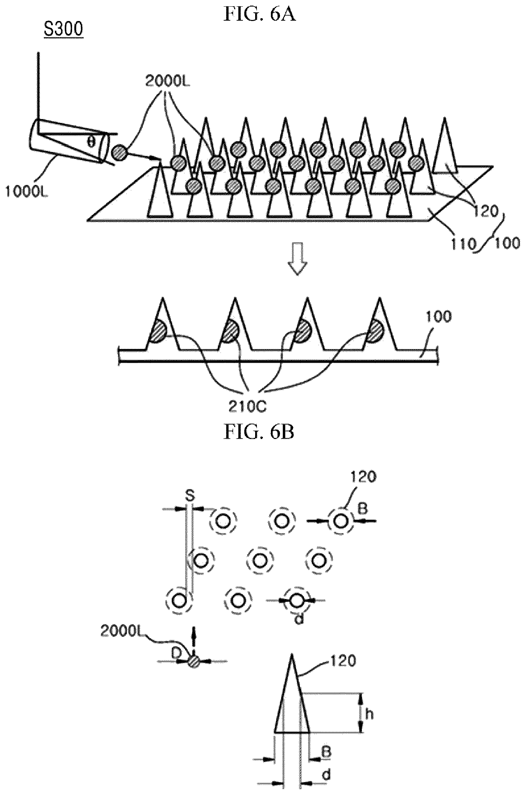

To provide the description with reference to FIG. 6 as an example, when the microstructures 120 are arranged in the form of the N X N matrix for example, the functional mixture may be coated on the plurality of microstructures 120 formed in a single microstructured thin film 100 rather than individually coating the functional mixture on each microstructure 120.

When the number of microstructures 120 formed in the single microstructured thin film 100 is increased to a certain level or more, the number of overlapping arrangement of microstructures 120 in a direction of spraying the functional mixture may also be increased. Therefore, the functional mixture may not reach the microstructure 120 disposed in regions overlapping each other in a straight line.

Therefore, depending on the number and arrangement of microstructures formed in the microstructured thin film, the functional mixture may be coated on all the plurality of microstructures formed in the microstructured thin film, or may be coated only on some of the microstructures formed in the microstructured thin film.

In this way, a different amount of the functional mixture may be coated on each microstructure. However, when a constant amount of the functional mixture is sprayed on the single microstructured thin film, a total amount of the functional mixture coated on the respective microstructured thin films may be the same as each other regardless of the number of microstructures coated with the functional mixture.

The functional mixture may include a mixed material including 0.01% to 100% by weight of nucleic acid having a molecular weight of 1kDa to 100MDa, and 0% to 99.99% by weight of a soluble functional material. Alternatively, the functional mixture may include the nucleic acid, or the functional mixture may be a mixture of the nucleic acid and the soluble functional material.

The nucleic acid may function to help a functional mixture solution go through a drying process to finally form the solid thin film.

In addition, the functional mixture may be a material which may be dissolved or decomposed in a living body. The functional mixture may consist of the following groups: deoxyribonucleic acid (DNA), ribonucleic acid (RNA), polydeoxyribonucleotide (PDRN), hyaluronic acid, alginic acid, pectin, carrageenan, chondroitin (sulfate), chondroitin sulfate, glycogen, dextran (sulfate), dextran, dextrin, chitosan, polylysine, collagen, gelatin, carboxymethyl chitin, fibrin, agarose, pullulan polylactide, polyglycolide (PGA), polylactide-glycolide copolymer (PLGA), hyaluronic acid, alginic acid, pectin, carrageenan, chondroitin (sulfate), dextran (sulfate), chitin, chitosan, polylysine, collagen, gelatin, polysaccharides such as carboxymethyl chitin, fibrin, agarose, and pullulan, proteins such as collagen, gelatin, and their hydrolysates, pullulan polyanhydride, polyortheoester, polyetherester, polycaprolactone, polyesteramide, poly (butyric acid), poly (valeric acid), polyurethane, polyacrylate, ethylene-vinyl acetate polymers, acrylic substituted cellulose acetate, nondegradable polyurethanes, polystyrene, polyvinyl chloride, polyvinyl fluoride, poly (vinyl imidazole), chlorosulphonatepolyolefins, polyethylene oxide, polyvinylpyrrolidone (PVP), polyvinyl alcohol, polyethylene glycol (PEG), polymethacrylate, polyacrylic acid, carboxyvinyl polymer, hydroxypropyl methylcellulose, ethylcellulose (EC), hydroxypropylcellulose (HPC), carboxymethylcellulose, cyclodextrin, and the copolymers of the monomers and celluloses that form these polymers.

### Step of coating mixture

Hereinafter, the description describes the step of coating the mixture (S300) in detail with reference to FIGS. 6 to 8.

FIG. 6 is a view showing the step of coating the mixture (S300) according to an embodiment of the present disclosure.

As shown in FIG. 6, according to an embodiment of the present disclosure, a functional mixture 2000L may have a droplet structure and be sprayed on the microstructure 120 to form a functional coating layer 210C.

Here, as shown in FIG. 6A, a spray angle of a droplet nozzle unit 1000L may preferably be adjusted for a droplet of the functional mixture 2000L not to reach a base thin film 110 to increase a coating rate in which the functional mixture 2000L is coated on the microstructure 120.

A spray angle θ of the droplet nozzle unit 1000L may preferably be adjusted based on a total height of the microstructure 120, that is, a region where the functional mixture 2000L is to be coated on the microstructure 120.

In addition, as shown in FIG. 6B, a maximum distance S between the adjacent microstructures 120 may preferably be smaller than a diameter D of the droplet of the functional mixture 2000L based on a cross section d of the microstructure that corresponds to a certain height h from a bottom B of the microstructure 120.

That is, the plurality of microstructures 120 may be formed for the droplet to be in contact with at least one microstructure 120 without escaping from the microstructured thin film as the droplet is moved when the droplet of the functional mixture 2000L is sprayed based on the certain height h of the microstructure 120. As a result, the droplet of the functional mixture 2000L that is sprayed from the droplet nozzle unit 1000L may be coated on the microstructure 120, and may be irregularly coated on an unspecified region of the microstructure 120.

According to an embodiment of the present disclosure, the droplet nozzle unit 1000L may spray the droplets several times by changing a spray position of the functional mixture 2000L, and adjust an amount of the functional mixture 2000L coated on the microstructure 120.

In addition, the droplet nozzle unit 1000L may coat the plurality of functional mixtures on the single microstructured thin film 100 by spraying various types of the functional mixtures on the same microstructure 120.

As described above, according to an embodiment of the present disclosure, firstly, the spray angle of the droplet nozzle unit 1000L may be adjusted to spray the functional mixture on the microstructure 120 while preventing the functional mixture from falling on the base thin film 110.

In addition, according to an embodiment of the present disclosure, secondarily, the maximum distance between the adjacent microstructures 120 may be formed to be smaller than the diameter D of the droplet of the functional mixture 2000L to prevent the sprayed functional mixture from escaping between the microstructures 120.

Through this configuration, the functional mixture sprayed from the droplet nozzle unit 1000L may be prevented from being coated on the base thin film 110 or escaping to the outside, all of the sprayed amount may substantially come into contact with the microstructure 120 formed on the base thin film 110, and at least a partial region of the microstructure 120 may thus be coated.

Therefore, the functional mixture to be delivered into the skin may be coated on the region of the microstructure 120 that is inserted into the skin, thus relatively accurately controlling the amount of the functional mixture delivered into the skin.

In addition, when coating a large amount of microstructured thin film, a certain amount of the functional mixture may be coated on each microstructured thin film by controlling the spray angle and the distance between the microstructures 120, thus quickly manufacturing the skin attachable thin film in which the functional mixture is coated on the microstructured thin film through a simple process.

In some examples, the droplet nozzle unit 1000L may be disposed at the certain height of the microstructure 120. In this case, the droplet nozzle unit 1000L may horizontally spray the functional mixture in a diameter direction of the microstructure, and the step of adjusting the spray angle may be omitted.

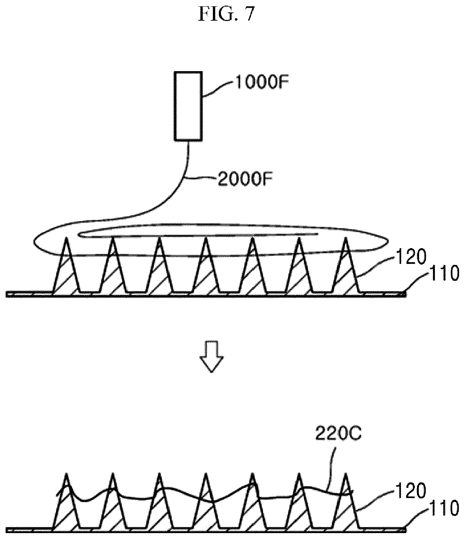

The description describes a step of coating the mixture (S300) in another example of the present disclosure with reference to FIG. 7.

In another example of the present disclosure, a functional mixture 2000F may have a fiber structure, and be sprayed on the microstructure 120 to form a functional coating layer 220C.

Here, a fiber nozzle unit 1000F may increase a coating rate of the functional mixture 2000F on the microstructure 120 by setting its spray angle to prevent a fiber of the functional mixture 2000F from reaching the base thin film 110.

In addition, an amount of functional mixture 2000F sprayed on the microstructure 120 may be adjusted by controlling the spray speed and movement speed of the fiber nozzle unit 1000F.

The fiber nozzle unit 1000F may preferably be moved by resetting a spray path of the fiber nozzle unit 1000F based on the region where the microstructure 120 is disposed, the height of the microstructure 120, or the like.

Here, the fiber nozzle unit 1000F may coat the plurality of functional mixtures with different functions on the single microstructured thin film 100 by spraying different types of functional mixtures on the same microstructure 120, for example, by sequentially spraying the mixture of the nucleic acid and the functional material and a mixture of deoxyribonucleic acid (DNA) and the functional material on one microstructure 120.

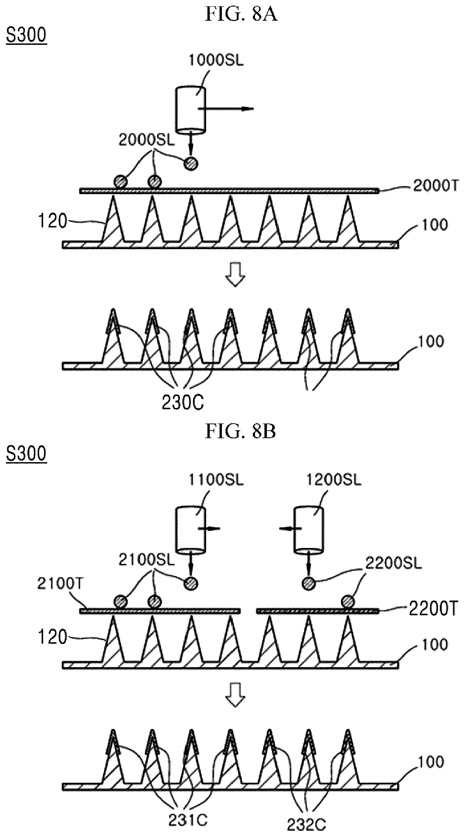

FIG. 8 is a view showing a step of coating the mixture (S300) in still another example of the present disclosure.

In this example, the step of coating the mixture (S300) may include a step of disposing a thin film mixture and a step of spraying a mixture droplet.

The step of disposing the thin film mixture may include a step of molding the thin film mixture and a step of aligning the thin film mixture.

In the step of molding the thin film mixture, the functional mixture may be a thin film-type thin film mixture 2000T. Here, the thin film mixture 2000T may have a thickness of several nm to several mm.

In the step of aligning the thin film mixture, the thin film mixture may be aligned for an upper part of the microstructure 120 and the thin film mixture 2000T to be in contact with each other, or the upper part of the microstructure 120 and the thin film mixture 2000T to be slightly spaced apart from each other.

In the step of spraying the mixture droplet, a single droplet spray unit 1000SL spraying a single droplet of a functional mixture 2000SL may spray the single droplet of the functional mixture 2000SL on a region in an upper part of the thin film mixture 2000T, which corresponds to the upper part of the microstructure 120.

Here, the thin film mixture 2000T or the single droplet of the functional mixture 2000SL may preferably be a solvent including water or a solution including the same functional mixture.

As the step of spraying the mixture droplet is performed, the region of the thin film mixture 2000T that is sprayed with the single droplet of the functional mixture 2000SL may be dissolved to cover an upper region of the microstructure 120, and the drying process may then be performed to coat a functional mixture 2000T and 2000SL on a surface of the microstructure 120, thereby forming a functional coating layer 230C.

Alternatively, as shown in FIG. 8B, the plurality of thin film mixtures 2000T and 2000SL may be disposed on the upper part of the microstructure 120, and materials included in each thin film mixture 2000T and 2000SL may include different materials as in the above-described example.

That is, a first thin film mixture 2100T may be a material made of mixing the nucleic acid with the functional material, and a second thin film mixture 2200T may be a material made of mixing the DNA with the functional material.

The first thin film mixture 2100T and the second thin film mixture 2200T may be disposed in different regions of the thin film 100.

Here, single droplet spray units 1100SL and 1200SL may spray a first single droplet 2100SL and a second single droplet 2200SL, which are the same functional mixture, on the respective thin film mixtures based on the regions where the first thin film mixture 2100T and the second thin film mixture 2200T are disposed to coat different functional coating layers 231C and 232C on the same microstructured thin film 100, thereby manufacturing the skin attachable thin film.

As a result, the plurality of functional mixtures may be coated on the single microstructured thin film 100 to thus enable injection of a medicine with a plurality of functions through the single skin attachable thin film.

### Step of adjusting angle

Referring back to FIG. 1B, the method of manufacturing a skin attachable thin film according to the second example of the present disclosure may include the step of adjusting the angle (S200) of adjusting the spray angle of the functional mixture 2000L coated on the microstructure 120 before the step of coating the mixture (S300) is performed.

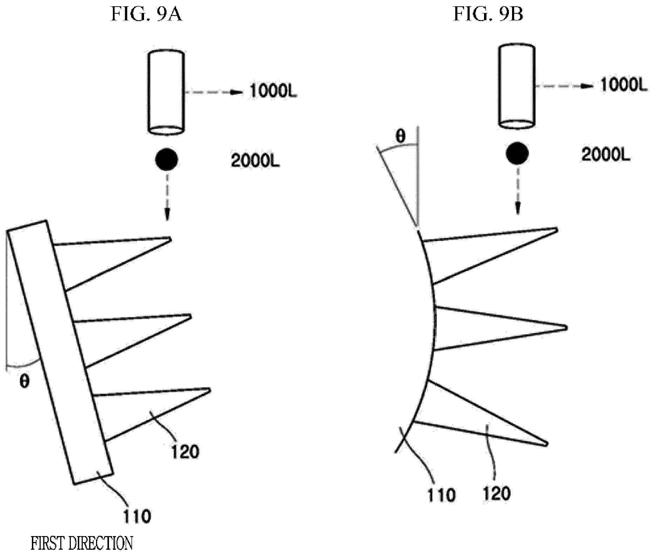

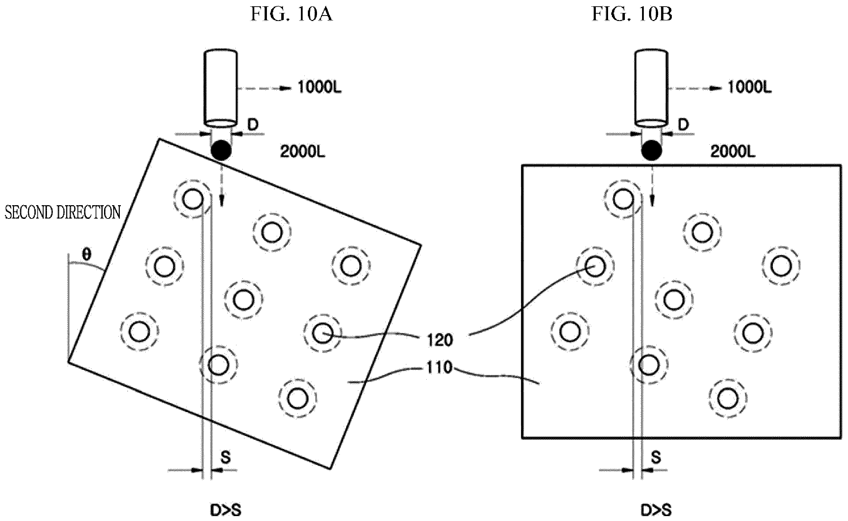

The step of adjusting the angle (S200) may be performed by dividing a direction in which the angle is adjusted into a first direction and a second direction, which is described with reference to FIGS. 9 and 10 as follows.

First, as shown in FIG. 9, in the step of adjusting the angle (S200), the functional mixture 2000L may be adjusted to an angle in the first direction, that is, a direction horizontal to a length direction of the base thin film 110 to prevent the functional mixture 2000L from being coated on the base thin film 110 and from escaping over the microstructure 120.

Alternatively, as shown in FIG. 10, in the step of adjusting the angle (S200), the angle may be adjusted to be the second direction to prevent the medicine from passing through a space between the microstructures 120 and escaping to the outside of the microstructured thin film when a droplet nozzle unit 1000L is moved along one side surface of the microstructured thin film and sprays the functional mixture 2000L.

That is, as shown in FIG. 10A, the angle of the microstructured thin film may be adjusted for the distance between the microstructure disposed closest to the droplet nozzle unit 1000L and the microstructure disposed farthest away therefrom based on the spray direction of the droplet nozzle unit 1000L (that is, the distance between the microstructures in a direction perpendicular to the spray direction), to be smaller than the diameter D of the droplet of the functional mixture 2000L.

In some examples, as shown in FIG. 10B, when formed on the base thin film, the microstructures may be disposed in a direction diagonal to the length direction or height direction of the base thin film in consideration of the diameter of the droplet. In this case, the step of adjusting the angle to be the second direction may be omitted.

To implement this example, the step of preparing the thin film (S100) according to the present disclosure may include a step of forming the microstructure of forming the microstructure 120 in which in the thin film including the base thin film 110 and the microstructure 120, the distance S between the two adjacent microstructures 120 in the direction perpendicular to the spray direction of the functional mixture 2000L is smaller than the diameter D of the droplet of the functional mixture 2000L, based on the cross section d of the microstructure 120 (see FIG. 6B) that corresponds to the certain height from the bottom of the microstructure 120, i.e., a lower part of the microstructure 120 that is connected to the base thin film 110.

That is, as shown in FIG. 10B, the distance between the two adjacent microstructures 120 in the direction perpendicular to the spray direction of the functional mixture 2000L may be disposed to be smaller than the diameter D of the droplet of the functional mixture 120, thereby stably coating the functional mixture 2000L on the microstructure 120 even though the step of adjusting the angle to be the second direction is omitted.

### Microstructure

Hereinafter, the description describes a microstructure formed in a microstructured thin film in detail.

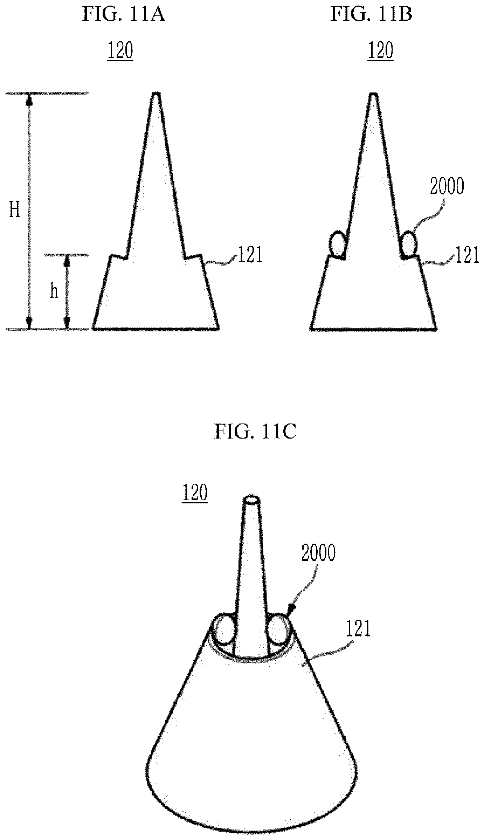

FIG. 11 is a view showing the microstructure according to an embodiment of the present disclosure.

Referring to FIG. 11A, a microstructure 120 may have one vertex, and include at least one ultramicrostructure 121 protruding therefrom. Here, a valley may be formed between the microstructure 120 and the ultramicrostructure 121, and this valley may allow a functional mixture 2000 to be coated on an upper part of the microstructure 120 without flowing down to a bottom of a base thin film.

Accordingly, when the microstructured thin film is attached to the skin, the functional mixture 2000 coated on the upper part of the microstructure 120 may be effectively injected into the skin. In addition, when the ultramicrostructure 121 is formed for a position of the valley formed between the microstructure 120 and the ultramicrostructure 121 to be disposed in the skin, most of regions coated with the functional mixture 2000 may be disposed in the skin, thus enabling more effective injection of the functional mixture 2000.

In addition, a height of the ultramicrostructure 121 may be adjusted based on an injection amount of the functional mixture. For example, a height h of at least one protruding ultramicrostructure may be 1/3 or more of a total height H of the microstructure 120 based on the base thin film.

The ultramicrostructure 121 may be formed along a sidewall of the microstructure 120, may have a plurality of stages, and may have a symmetrical structure. For example, as shown in FIG. 11A, the ultramicrostructure 121 may have two stages, and may have the symmetrical structure.

Referring to FIGS. 11A and 11C, the ultramicrostructure 121 may have a structure such as an inclined structure inclined toward the center of the microstructure or a structure in which an outer edge protrudes more than the center to prevent the functional mixture 2000 from flowing down along a surface of the microstructure 120 when the functional mixture 2000 is coated thereon. For example, referring back to FIG. 11A, the microstructure 121 may have two stages to prevent the functional mixture 2000 from flowing down.

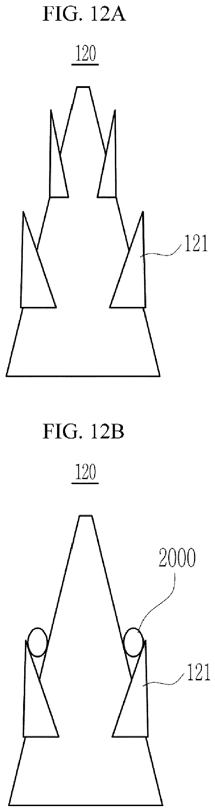

FIG. 12 is a view showing a microstructure in another example of the present disclosure.

In describing the microstructure 120, the same content as that of the structure described above is omitted.

Referring to FIGS. 12A and 12B, at least one ultramicrostructure 121 may protrude from the microstructure 120. In comparison with FIG. 11, the ultramicrostructure 121 of the microstructure 120 may have a protruding cone-shaped structure. However, even though the ultramicrostructure 121 protrudes, the ultramicrostructure 121 may not be higher than the microstructure 120.

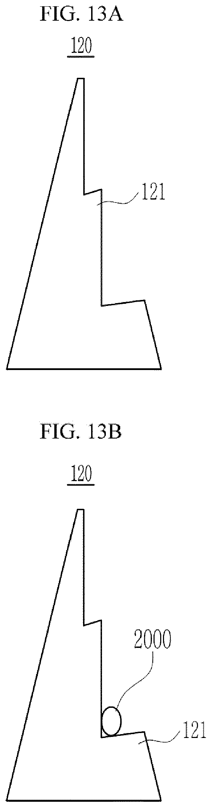

FIG. 13 is a view showing a microstructure in still another example of the present disclosure.

Referring to FIGS. 13A and 13B, the microstructure 120 may have at least one ultramicrostructure 121 formed by molding or processing to have a step structure. That is, unlike the microstructure described above, the ultramicrostructure 121 may have an asymmetric structure, and have a step having a plurality of stages.

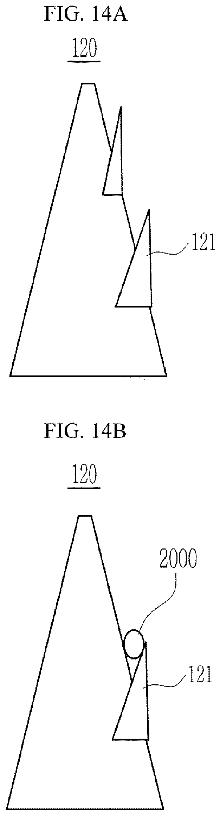

FIG. 14 is a view showing a microstructure in yet another example of the present disclosure.

In describing the microstructure 120, the same content as that of the structure described above is omitted.

Referring to FIG. 14, at least one ultramicrostructure 121 may protrude from the microstructure 120. In comparison with the microstructure of FIG. 11 or FIG. 12, the ultramicrostructure 121 of the microstructure 120 may have an asymmetrical protruding structure.

### Microstructured thin film

Hereinafter, the description describes the microstructured thin film according to the present disclosure.

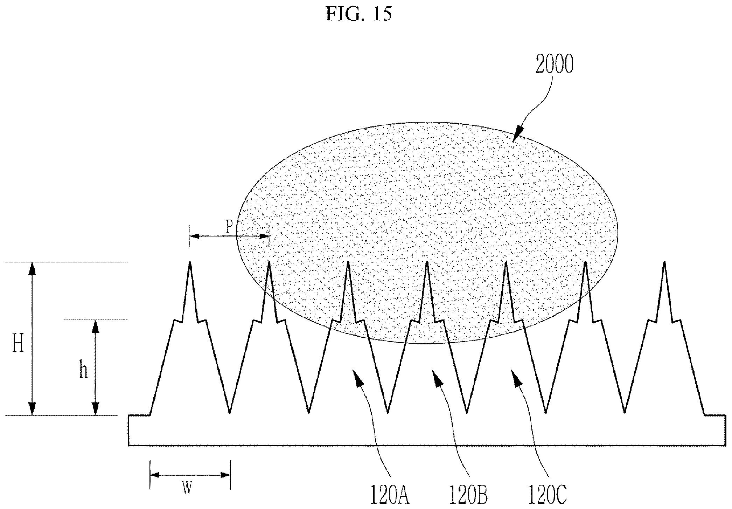

FIG. 15 is a view showing the microstructured thin film according to an embodiment of the present disclosure.

Referring to FIG. 15, a microstructured thin film 100 (which is the same as shown in FIG. 6) may have a distance P between microstructures 120A, 120B and 120C that is measured based on a vertex of the microstructure, which is longer than a length W of a lower surface of the microstructure, and shorter than a height H of the microstructure that is measured based on the base thin film to prevent the functional mixture 2000 from being in contact with the base thin film 110 (which is the same as shown in FIG. 6) when the functional mixture 2000 is coated thereon.

That is, the distance P between the microstructures (that is, the distance between the microstructures 120A and 120B or the distance between the microstructures 120B and 120C) may be longer than the length W of the lower surface of the microstructure and shorter than the height H of the microstructure, thus preventing the functional mixture 2000 from being in contact with the base thin film when the functional mixture 2000 is coated on the microstructure. Here, the ultramicrostructure of the microstructure may protrude to prevent the functional mixture from being in contact with the base thin film.

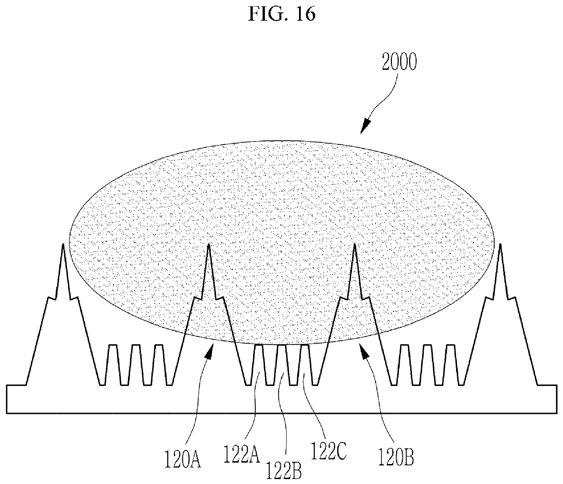

FIG. 16 is a view showing a microstructured thin film in another example of the present disclosure.

Referring to FIG. 16, the microstructured thin film 100 (which is the same as shown in FIG. 6) may include a plurality of nanomicro structures each having a size smaller than that of the microstructure and disposed between one or more microstructures to prevent the functional mixture 2000 from being in contact with the base thin film 110 (which is the same as shown in FIG. 6) when the functional mixture 2000 is coated on the microstructure.

For example, as shown in FIG. 16, a plurality of nanomicro structures 122A, 122B, and 122C each having the size smaller than that of the microstructure may be disposed between the microstructures 120A and 120B. Therefore, the nanomicro structure may prevent the functional mixture from being in contact with the base thin film when the functional mixture is coated on the microstructure.

### Another method of manufacturing thin film attached to skin

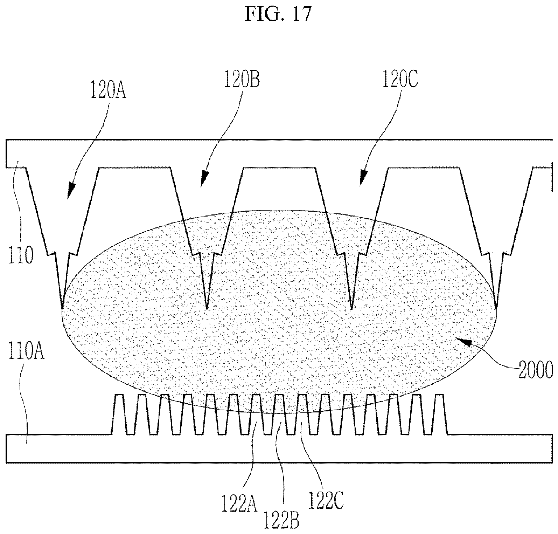

FIG. 17 is a view for explaining a method of manufacturing a skin attachable thin film in a third example of the present disclosure.

In describing the method of manufacturing a skin attachable thin film, the same content as the above is omitted.

The method of manufacturing a skin attachable thin film in the third example of the present disclosure may include a step of preparing the thin film and a step of coating the mixture like the manufacturing method in the other examples.

In the step of preparing the thin film of the third example, a nanomicro thin film including a plurality of nanomicro structures each having a size smaller than that of the microstructure may be prepared. In the step of coating the mixture, the functional mixture may be coated on the nanomicro structure, and the microstructured thin film and the nanomicro thin film may be disposed to face each other to thus bring the functional mixture into contact with the microstructure. In this way, the functional mixture may be coated on the microstructure excluding the region of the base film of the microstructured thin film.

For example, referring to FIG. 17, according to the method of manufacturing a skin attachable thin film, a functional mixture 2000 may be coated on a microstructure 120A, 120B, or 120C by excluding the region of the base thin film 110 (which is the same as in FIG. 6) of the microstructured thin film 100 (which is the same as in FIG. 6) by preparing a nanomicro thin film 110A, which is a separate structure from the base thin film 110 (which is the same as in FIG. 6) including the microstructure 120A, 120B or 120C, and includes a plurality of nanomicro structures 122A, 122B, or 122C smaller than the microstructure, coating the functional mixture 2000 on the nanomicro structure 122A, 122B or 122C, and allowing the nanomicro thin film 110A and the microstructured thin film 100 (which is the same as shown in FIG. 6) to face each other to thus allow the functional mixture 2000 to be in contact with the microstructure 120A, 120B or 120C.

As described above, the present disclosure is not limited to the above-mentioned specific embodiments, and may be variously modified by those skilled in the art to which the present disclosure pertains without departing from the scope and spirit of the present disclosure as disclosed in the accompanying claims. These modifications should also be understood to fall within the scope of the present disclosure.

## Claims

1. A method of manufacturing a skin attachable thin film coated with a functional mixture, the method comprising:
preparing a microstructured thin film including a base thin film and at least one microstructure protruding from the base thin film; and
coating a functional mixture on the microstructure excluding a region of the base film of the microstructured thin film,
wherein in the coating of the mixture, the functional mixture is irregularly coated on the at least one microstructure.

2. The method of claim 1, further comprising
adjusting a spray angle of the functional mixture to be coated on the microstructure before the functional mixture is coated.

3. The method of claim 1 or 2, further comprising
coating a binder material on the microstructure for increasing an adhesive strength between the functional mixture and the microstructure, before the functional mixture is coated.

4. The method of claim 1 or 2, wherein
in the coating of the mixture, the functional mixture has a droplet structure and is sprayed on the microstructure.

5. The method of claim 1 or 2, wherein
in the coating of the mixture, the functional mixture has a fiber structure and is sprayed on the microstructure.

6. The method of claim 1 or 2, wherein
the microstructured thin film includes any one of an insoluble material, a low solubility material, and a biodegradable material, or a mixture of the insoluble material, the low solubility material, and the biodegradable material.

7. The method of claim 6, wherein
the preparing of the microstructured thin film includes:
preparing a mold having a structure corresponding to a shape of the microstructure to be formed;
coating a thin film material, which is a material of the microstructured thin film, on the mold;
molding the microstructured thin film by pressing and deforming the thin film material to have the structure formed in the mold as a pressing unit is moved toward the mold; and
acquiring the microstructured thin film by separating the microstructured thin film from the mold.

8. The method of claim 6, wherein
the preparing of the microstructured thin film includes:
preparing a mold having a structure corresponding to a shape of the microstructure to be formed;
coating a thin film material, which is a material of the microstructured thin film, on the mold;
molding the microstructured thin film as the thin film material coated on the mold is dried; and
acquiring the microstructured thin film by separating the microstructured thin film from the mold.

9. The method of claim 6, wherein
the preparing of the microstructured thin film includes:
preparing a mold having a structure corresponding to a shape of the microstructure to be formed;
coating a thin film material, which is a material of the microstructured thin film, on the mold;
molding the microstructured thin film by curing the thin film material coated on the mold by emitting heat or light to the thin film material; and
acquiring the microstructured thin film by separating the microstructured thin film from the mold,
wherein in the molding the microstructured thin film, light emitted to the thin film material is any one of ultraviolet light, visible light, and infrared light.

10. The method of claim 6, wherein
the preparing of the microstructured thin film includes:
manufacturing the thin film plate having a flat plate shape by using a thin film material, which is a material of the microstructured thin film; and
cutting and bending a region of the thin film plate that is pressed by a concave-convex part as a pressing unit disposed above the thin film plate and having the concave-convex part formed toward the thin film plate is moved toward the thin film plate,
wherein in the cutting and bending, the region of the thin film plate that is pressed and bent by the concave-convex part is the microstructure.

11. The method of claim 1 or 2, wherein
the preparing of the microstructured thin film includes forming the microstructure for a distance between the two adjacent microstructures in a direction perpendicular to a spray direction of the functional mixture to be smaller than a diameter of a droplet of the functional mixture, based on a cross section of the microstructure that corresponds to a certain height from a lower part of the microstructure that is connected to the base thin film.

12. A skin attachable thin film, the film comprising:
a microstructured thin film including a base thin film and at least one microstructure protruding from the base thin film;
a coating layer having a functional mixture coated on the at least one microstructure, and irregularly coated on at least a partial region of the microstructure excluding a region of the base film; and
a binder material coated on a surface of the microstructure to increase an adhesive strength between the functional mixture and the microstructure.

13. A skin attachable thin film, wherein the film is a microstructured thin film having a functional mixture sprayed and coated thereon,
the microstructured thin film includes a base thin film and a plurality of microstructures protruding from the base thin film, and
a distance between the two adjacent microstructures in a direction perpendicular to a spray direction of the functional mixture is smaller than a diameter of a droplet of the functional mixture, based on a cross section of the microstructure that corresponds to a certain height from each bottom of the plurality of microstructures.

14. The film of claim 12 or 13, wherein
the microstructure has
one vertex, and includes at least one ultramicrostructure protruding therefrom.

15. The film of claim 14, wherein
a height of the at least one protruding ultramicrostructure is
1/3 or more of the height of the microstructure based on the base thin film.

16. The film of claim 14, wherein
the ultramicrostructure has
a structure for preventing the functional mixture from flowing down along a surface of the microstructure when the functional mixture is coated on the microstructure.

17. The film of claim 14, wherein
the microstructured thin film includes
at least one microstructure to prevent the functional mixture from being in contact with the base thin film when the functional mixture is coated on the microstructure.

18. The film of claim 14, wherein
the microstructured thin film has
a distance between the microstructures that is measured based on the vertex of the microstructure, which is longer than a length of a lower surface of the microstructure, and shorter than the height of the microstructure that is measured based on the base thin film to prevent the functional mixture from being in contact with the base thin film when the functional mixture is coated on the microstructure.

19. The film of claim 14, wherein
the microstructured thin film includes
a plurality of nanomicro structures each having a size smaller than that of the microstructure and disposed between the one or more microstructures to prevent the functional mixture from being in contact with the base thin film when the functional mixture is coated on the microstructure.

20. The method of claim 1, wherein
in the preparing the thin film,
a nanomicro thin film including a plurality of nanomicro structures each having a size smaller than that of the microstructure is prepared, and
in the coating the mixture,
the functional mixture is coated on the microstructure excluding the region of the base film of the microstructured thin film by coating the functional mixture on the nanomicro structure, and disposing the microstructured thin film and the nanomicro thin film to face each other to thus bring the functional mixture into contact with the microstructure.
